# FASCICULE DE BREVET EUROPEEN

(11) **EP 0 536 064 B1**
(45) Date de publication et mention de la délivrance du brevet: **09.08.1995**
(21) Numéro de dépôt: 92420335.9
(22) Date de dépôt: 28.09.1992
(51) Int. Cl.: C07C 51/14, C07C 67/38

(54) **Procédé de préparation de l'acide adipique par hydrocarboxylation d'acides penteniques**
Verfahren zur Herstellung von Adipinsäure durch Hydroxycarboxylierung der Pentensäuren
Process for preparing adipic acid by hydrocarboxylation of pentenic acids

(30) Priorité: 03.10.1991 FR 9112392
(43) Date de publication de la demande: 07.04.1993
(73) Titulaire: RHONE-POULENC CHIMIE, 92408 Courbevoie Cédex (FR)
(72) Inventeur: Denis, Philippe, F-69150 Decines (FR); Grosselin, Jean-Michel, F-69340 Francheville (FR); Metz, François, F-69390 Vernaison (FR)
(74) Mandataire: Vignally, Noel

(56) Documents cités:
- US-A- 3 579 551
- CATALYSIS REWIEWS vol. 23, no. 1 &2, 1981, NEW YORK US pages 89 - 105 D.FORSTER ET AL. 'MECHANISTIC PATHWAYS IN THE CATALYSIS OF OLEFIN HYDROCARBOXYLATION BY RHODIUM, IRIDIUM, AND COBALT COMPLEXES'

## Description

La présente invention concerne un procédé de préparation de l'acide adipique par hydrocarboxylation d'acides penténiques, c'est-à-dire par réaction de l'eau et de l'oxyde de carbone sur au moins un acide penténique.

Dans le brevet américain 3,579,551 il a été proposé un procédé de préparation d'acides carboxyliques par réaction de composés à insaturation éthylénique avec de l'oxyde de carbone et de l'eau, en présence d'une composition catalytique comprenant essentiellement des composés ou complexes de l'iridium, et un promoteur iodé. Les composés à insaturation éthylénique sont convertis sélectivement en acides carboxyliques (linéaires et branchés) par la réaction en cause conduite, de préférence en phase liquide, à une température comprise entre 50 et 300°C (de préférence entre 125 et 225°C), sous des pressions partielles d'oxyde de carbone avantageusement comprises entre 5 et 3000 psia voire, entre 25 et 1000 psia.

N'importe quelle source d'iridium paraît pouvoir être utilisée et diverses sources de promoteurs iodés sont mentionnées ; le rapport atomique I/Ir peut varier dans de larges limites (1:1 à 2500:1) et de préférence entre 3:1 et 300:1.

Le milieu réactionnel liquide peut renfermer n'importe quel solvant compatible avec le système catalytique, les acides monocarboxyliques en C₂-C₂₀ étant les solvants préférés.

L'exemple 1 réalisé au départ de propylène montre qu'un tel système favorise la formation d'acides carboxyliques branchés (isobutyrique).

L'exemple 19 réalise au départ d'héxène-1 confirme l'importance de la proportion d'acides carboxyliques branchés ainsi obtenus.

Cet inconvénient (manque de sélectivité en acide carboxylique linéaire) n'a pas échappé au titulaire du brevet américain précité. En effet, dans le brevet américain n° 3,816,489, il est proposé de mettre en oeuvre la réaction en cause avec un rapport atomique I/Ir compris entre 3:1 et 100:1 pour obtenir des acides carboxyliques terminaux de manière prépondérante.

Si l'intérêt de principe de telles techniques n'est pas remis en cause, dans le cas de matières premières choisies parmi les composés à insaturation oléfinique non fonctionnalisés et en particulier dans le cas des oléfines elles-mêmes, la transposition de ces techniques à des matières premières comportant en plus de l'insaturation éthylénique, une fonction réactive dans les conditions de la réaction en cause, se heurte à de nombreuses difficultés.

En particulier, les premières tentatives de transpositions réalisées par la Demanderesse au départ d'acides penténiques se sont soldées au moins par un échec partiel, des réactions différentes de celle spécifiquement visée se produisant au détriment voire en remplacement total de celle-ci en raison de la présence d'une fonction -COOH sur la matière première à insaturation éthylénique.

Dans un tel contexte il serait manifestement souhaitable de pouvoir disposer d'un procédé offrant simultanément de bonnes performances dans la réaction de carbonylation visée et une sélectivité appréciable en acide adipique.

La présente invention a donc pour objet un procédé de préparation de l'acide adipique par réaction de l'eau et de l'oxyde de carbone sur au moins un acide pentènique, en présence d'un catalyseur à base d'iridium et d'au moins un promoteur iodé, à une température élevée, sous une pression supérieure à la pression atmosphérique, dans au moins un solvant choisi dans le groupe constitué par les hydrocarbures saturés aliphatiques ou cycloaliphatiques et leurs dérivés halogènés, les hydrocarbures aromatiques et leurs dérivés halogénés, et les éthers aliphatiques, aromatiques ou mixtes, le rapport atomique I/Ir étant inférieur à 20.

Par acide penténique, on entend dans le cadre de la présente invention l'acide pentène-2 oïque, l'acide pentène-3 oïque, l'acide pentène-4 oïque et leurs mélanges.

L'acide pentène-4 oïque conduit à de bons résultats, mais reste peu disponible.

L'acide pentène-3 oïque pris isolément ou en mélange avec ses isomères est plus particulièrement approprié compte-tenu de son accessibilité et des résultats satisfaisants auxquels il conduit dans le cadre du présent procédé.

Le procédé selon la présente invention nécessite la présence d'un catalyseur à base d'iridium. Diverses sources d'iridium sont susceptibles d'être mises en oeuvre.

A titre d'exemple de sources d'iridium susceptibles de convenir à la mise en oeuvre du présent procédé on peut citer :
Ir métallique ; Ir O₂ ; Ir₂ O₃ ;
Ir Cl₃ ; Ir Cl₃ , 3 H₂O ;
Ir Br₃ ; Ir Br₃ , 3H₂O ;
Ir I₃ ;
Ir₂(CO)₄Cl₂ ; Ir₂(CO)₄I₂ ;
Ir₂(CO)₈ ; Ir₄(CO)₁₂ ;
Ir₂(CO)[P(C₆H₅)₃]₂ I ;
Ir(CO)[P(C₆H₅)₃]₂ Cl ;
Ir[P(C₆H₅)₃]₃I ;
HIr[P(C₆H₅)₃]₃(CO) ;
Ir(acac)(CO)₂
[IrCl(Cod)]₂
(Cod : cyclooctadiène-1,5 ; acac : acétylacétonate)
Conviennent plus particulièrement à la mise en oeuvre du présent procédé : [IrCl(Cod)]₂ ; Ir₄(CO)₁₂ et Ir(acac)(CO)₂.

La quantité d'iridium à mettre en oeuvre peut varier dans de larges limites.

En général, une quantité exprimée en mole d'iridium métallique par litre de milieu réactionnel comprise entre 10⁻³ et 10⁻¹ conduit à des résultats satisfaisants. Des quantités inférieures peuvent être mises en oeuvre ; on observe toutefois que la vitesse de réaction est faible. Des quantités supérieures n'ont d'inconvénients qu'au plan économique.

De préférence, la concentration d'iridium est comprise entre 5.10⁻³ et 10⁻² (inclus) mol/l.

Par promoteur iodé on entend dans le cadre du présent procédé HI et les composés organoiodés capables de générer HI dans les conditions réactionnelles et, en particulier les iodures d'alkyles en C₁-C₁₀. De préférence, on recourt à HI.

Selon une caractéristique essentielle du présent procédé, la quantité de promoteur iodé à mettre en oeuvre est telle que le rapport molaire I/Ir soit inférieur à 20. Il s'avère souhaitable que ce rapport soit supérieur ou égal à 0,1. De préférence, le rapport molaire I/Ir est inférieur à 10. Pour une bonne mise en oeuvre de l'invention, il sera compris entre 1 et 5 (inclus).

La présence d'eau est indispensable à la conduite du procédé selon la présente invention. En général, la quantité d'eau à mettre en oeuvre est telle que le rapport molaire eau/acide(s) penténique(s) soit compris entre 1 et 10 (inclus).

Une quantité inférieure présente l'inconvénient de limiter la conversion. Une quantité supérieure n'est pas souhaitable en raison de la perte d'activité catalytique observée.

Selon une autre des caractéristiques essentielles de la présente invention, la réaction est mise en oeuvre dans au moins un solvant choisi dans le groupe constitué par les hydrocarbures saturés aliphatiques ou cycloaliphatiques et leurs dérivés halogénés, les hydrocarbures aromatiques et leurs dérivés halogénés, et les éthers aliphatiques, aromatiques ou mixtes.

La nature précise du solvant choisi dans le groupe précité n'est pas critique dans le cadre du présent procédé, pour autant que celui-ci soit à l'état liquide dans les conditions réactionnelles.

A titre d'exemples de tels solvants on peut citer : le benzène, le toluène, le chlorobenzène, le chlorure de méthylène, le dichloro-1,2 éthane, l'hexane, le cyclohexane et le diphényléther.

La quantité de solvant présente dans le milieu réactionnel peut varier dans de larges limites, par exemple de 10 à 99 % (inclus) en volume du milieu réactionnel. De préférence cette quantité est comprise entre 30 et 90 % en volume (inclus).

Comme indiqué en tête du présent mémoire, la réaction est conduite sous une pression supérieure à la pression atmosphérique et en présence d'oxyde de carbone.

On peut utiliser de l'oxyde de carbone sensiblement pur ou de qualité technique, tel qu'il se présente dans le commerce.

La réaction est conduite de préférence en phase liquide, la pression totale pouvant varier dans de larges limites et la température de réaction étant généralement comprise entre 100 et 240°C et, de préférence entre 160 et 190°C.

La pression partielle de l'oxyde de carbone est généralement comprise entre 1 et 250 bars et pour une bonne mise en oeuvre du procédé selon l'invention, elle sera comprise entre 2 et 100 bar.

Le milieu réactionnel renferme au moins un solvant choisi dans le groupe défini ci-avant, de l'eau, une ou des sources d'iridium, un ou des promoteurs iodés et, le cas échéant tout ou partie du ou des acides penténiques engagés et des produits de la réaction.

En fin de réaction ou du temps imparti à celle-ci, on sépare l'acide adipique par tout moyen approprié par exemple par cristallisation et filtration.

Les exemples ci-après illustrent la présente invention.

### EXEMPLE 1 :

Dans une ampoule de verre préalablement purgée à l'argon, on introduit :
30 mg (0,1 mmol) d'iridium sous forme de Ir₄(CO)₁₂
0,045 g (0,2 mmol) de HI sous forme d'une solution dans le chlorobenzène (1 ml)
0,54 g ( 30 mmol) d'eau
2 g ( 20 mmol) d'acide pentène-3 oïque
9 cm³ de chlorobenzène
L'ampoule est placée dans un autoclave de 125 ml.

L'autoclave est fermé hermétiquement, placé dans un four agité et raccordé à l'alimentation de gaz sous pression. On admet à froid 2 bar de CO et on chauffe à 175°C en 20 minutes. Lorsque cette température est atteinte, on régule la pression à 20 bar.

Après une durée de réaction de 30 minutes, l'agitation et le chauffage sont arrêtés ; l'autoclave est alors refroidi et dégazé.

La solution réactionnelle est analysée par chromatographie en phase gazeuse et par chromatographie liquide haute performance.

Les quantités de produits formés (rendement molaire par rapport à l'acide pentène-3 oïque chargé) sont les suivantes : 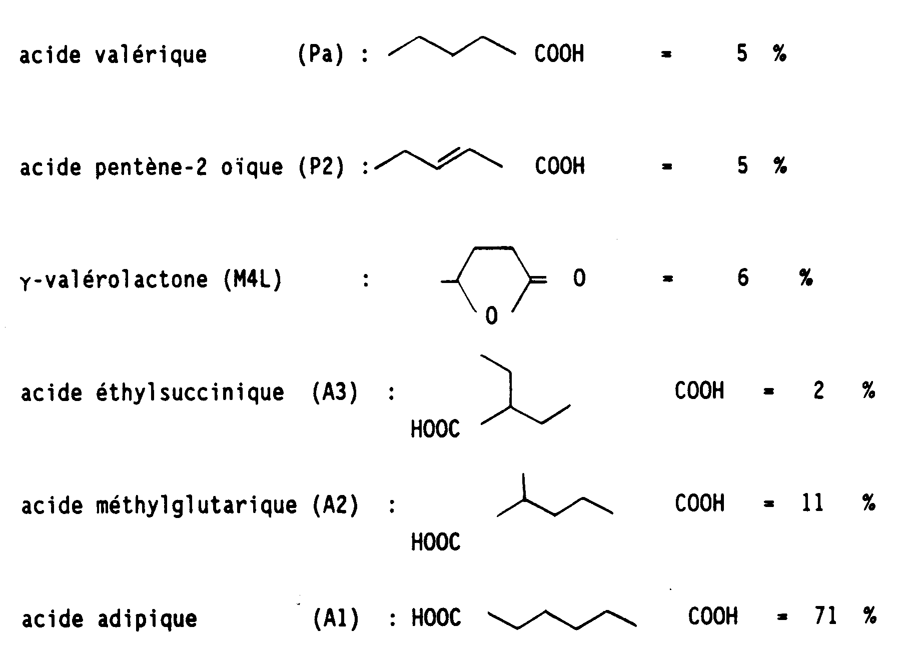
Le taux de linéarité (L) est de 84 %
Le taux de transformation de l'acide pentène-3 oïque (TT) est de 93 %.

### EXEMPLE 2 :

On reproduit l'exemple 1 ci-avant à ceci près que l'on engage HI sous forme d'une solution (0,2 mmol) dans l'acide acétique (1 ml).

Toutes conditions égales par ailleurs on obtient sensiblement les mêmes résultats à ceci près que l'on ne détecte plus la présence d'acide pentène-2 oïque et que le rendement molaire en γ-valérolactone par rapport à l'acide pentène-3 oïque chargé (M4L) est de 12,5 %.

### EXEMPLES 3 à 6 ; essai comparatif (a) :

On reproduit l'exemple 1 ci-avant en utilisant des solvants de nature différente et en utilisant une solution de HI (0,2 mmol) dans l'acide acétique. Les conditions particulières ainsi que les résultats obtenus sont rassemblés dans le tableau I ci-après dans lequel
- t désigne la durée de l'essai en température.

**TABLEAU I**

| Réf. | Solvant (nature) | t mn | TT % | A1 % | L % | M4L % |
|---|---|---|---|---|---|---|
| 2 | chlorobenzène | 30 | 93 | 71 | 84 | 12,5 |
| 3(*) | chlorure de méthylène | 35 | 100 | 70 | 84 | 10,0 |
| 4 | toluène | 35 | 95 | 75 | 84 | 7,5 |
| 5 | cyclohexane | 30 | 96 | 68 | 80 | 8 |
| 6 | diphényléther | 40 | 100 | 63 | 78 | 12 |
| a | eau/toluène (50/50 vol.) | 120 | 18 | ε | ND | 40 |

| | | | | | | |
|---|---|---|---|---|---|---|
| (*) Dans cet exemple la pression totale en température est de 30 bar. | | | | | | |
| ND : non déterminée | | | | | | |

Dans l'essai témoin (a) on note la formation d'une quantité prépondérante d'acide pentanoïque. [PA (%) = 40].

### EXEMPLES 7 à 8 ; essai comparatif (b) :

Selon le mode opératoire décrit pour l'exemple 1, on réalise une série d'essais dans le toluène et en modifiant la quantité d'HI chargée.

Les conditions particulières ainsi que les résultats obtenus toutes conditions égales par ailleurs sont rassemblés dans le tableau (II) ci-après, dans lequel les conventions utilisées sont les mêmes que pour l'exemple 1 et t désigne la durée de l'essai en température :

**TABLEAU II**

| Réf. | HI/Ir | t mn | TT % | A1 % | L% | M4L % |
|---|---|---|---|---|---|---|
| a | 0 | 90 | 0 | - | - | - |
| 1 | 2 | 30 | 93 | 71 | 84 | 6 |
| 7 | 5 | 35 | 99 | 44 | 78 | 33 |
| 8 | 9 | 85 | 78 | 21 | 81 | 65 |

## Revendications

1. Procédé de préparation de l'acide adipique par réaction de l'eau et de l'oxyde de carbone sur au moins un acide pentènique, en présence d'un catalyseur à base d'iridium et d'au moins un promoteur iodé à une température élevée, sous une pression supérieure à la pression atmosphérique, dans au moins un solvant choisi dans le groupe constitué par les hydrocarbures saturés aliphatiques ou cycloaliphatiques et leurs dérivés halogénés, les hydrocarbures aromatiques et leurs dérivés halogénés, et les éthers aliphatiques, aromatiques ou mixtes, le rapport atomique I/Ir étant inférieur à 20.

2. Procédé selon la revendication 1, caractérisé en ce que l'acide pentènique est choisi parmi l'acide pentène-3 oïque, l'acide pentène-2 oïque, l'acide pentène-4 oïque et leurs mélanges.

3. Procédé selon l'une quelconque des revendications précédentes, caractérisé en ce que le rapport molaire eau/acide(s) pentènique(s) est inférieur ou égal à 10.

4. Procédé selon l'une quelconque des revendications précédentes, caractérisé en ce que le solvant est choisi parmi le benzène, le toluène, le chlorobenzène, l'hexane, le cyclohexane, le chlorure de méthylène, le dichloro-1,2 éthane et le diphényléther.

5. Procédé selon l'une quelconque des revendications précédentes, caractérisé en ce que le solvant représente au minimum 10 % du volume du milieu réactionnel.

6. Procédé selon la revendication 5, caractérisé en ce que le solvant représente de 30 à 90 % (inclus) du volume du milieu réactionnel.

7. Procédé selon l'une quelconque des revendications précécentes, caractérisé en ce que la concentration en iridium dans le milieu réactionnel est comprise entre 10⁻³ et 10⁻¹ mol/l (inclus).

8. Procédé selon l'une quelconque des revendications précédentes, caractérisé en ce que la température de réaction est comprise entre 100 et 240°C et, de préférence entre 160 et 190°C.

9. Procédé selon l'une quelconque des revendications précédentes, caractérisé en ce que la pression partielle de l'oxyde de carbone est comprise entre 1 et 250 bar et, de préférence entre 2 et 100 bar.

10. Procédé selon l'une quelconque des revendications précédentes, caractérisé en ce que le rapport atomique I/Ir est inférieur à 10.

11. Procédé selon l'une quelconque des revendications précédentes, caractérisé en ce que le rapport atomique I/Ir est supérieur ou égal à 0,1.

12. Procédé selon l'une quelconque des revendications précédentes, caractérisé en ce que le rapport atomique I/Ir est compris entre 1 et 5 (inclus).

13. Procédé selon l'une quelconque des revendications précédentes, caractérisé en ce qu'on utilise HI comme promoteur iodé.

## Claims

1. Process for the preparation of adipic acid by reaction of water and carbon monoxide with at least one pentenic acid, in the presence of an iridium-based catalyst and of at least one iodinated promoter, at a high temperature, at a pressure greater than atmospheric pressure, in at least one solvent chosen from the group comprising saturated aliphatic or cycloaliphatic hydrocarbons and their halogenated derivatives, aromatic hydrocarbons and their halogenated derivatives and aliphatic, aromatic or mixed ethers, the I/Ir atomic ratio being less than 20.

2. Process according to claim 1, characterized in that the pentenic acid is chosen from penten-3-oic acid, penten-2-oic acid, penten-4-oic acid and their mixtures.

3. Process according to either of the preceding claims, characterized in that the water/pentenic acid(s) molar ratio is less than or equal to 10.

4. Process according to any one of the preceding claims, characterized in that the solvent is chosen from benzene, toluene, chlorobenzene, hexane, cyclohexane, methylene chloride, 1,2-dichloroethane and diphenyl ether.

5. Process according to any one of the preceding claims, characterized in that the solvent represents at least 10 % of the volume of the reaction mixture.

6. Process according to claim 5, characterized in that the solvent represents from 30 to 90 % (inclusive) of the volume of the reaction mixture.

7. Process according to any one of the preceding claims, characterized in that the concentration of iridium in the reaction mixture is between 10⁻³ and 10⁻¹ mol/l (inclusive).

8. Process according to any one of the preceding claims, characterized in that the reaction temperature is between 100 and 240°C and preferably between 160 and 190°C.

9. Process according to any one of the preceding claims, characterized in that the carbon monoxide partial pressure is between 1 and 250 bar and preferably between 2 and 100 bar.

10. Process according to any one of the preceding claims, characterized in that the I/Ir atomic ratio is less than 10.

11. Process according to any one of the preceding claims, characterized in that the I/Ir atomic ratio is greater than or equal to 0.1.

12. Process according to any one of the preceding claims, characterized in that the I/Ir atomic ratio is between 1 and 5 (inclusive).

13. Process according to any one of the preceding claims, characterized in that HI is used as iodinated promoter.

## Patentansprüche

1. Verfahren zur Herstellung von Adipinsäure durch Reaktion von Wasser und Kohlenmonoxid mit mindestens einer Pentensäure in Anwesenheit eines Katalysators auf der Basis von Iridium und eines Iod-Promotors, bei erhöhter Temperatur, unter einem höheren Druck als dem atmosphärischen Druck und in mindestens einem Lösungsmittel, gewählt aus der Gruppe, die von den gesättigten aliphatischen oder cycloaliphatischen Kohlenwasserstoffen und ihren halogenierten Derivaten, den aromatischen Kohlenwasserstoffen und ihren halogenierten Derivaten und den aliphatischen, aromatischen oder gemischten Ethern gebildet wird, wobei das atomare Verhältnis I/Ir unter 20 liegt.

2. Verfahren nach Anspruch 1, dadurch gekennzeichnet, daß die Pentensäure unter Penten-3-säure, Penten-2-säure, Penten-4-säure und ihren Mischungen ausgewählt wird.

3. Verfahren nach irgendeinem der vorstehenden Ansprüche, dadurch gekennzeichnet, daß das molare Verhältnis Wasser/Pentensäure(n) niedriger als oder gleich 10 beträgt.

4. Verfahren nach irgendeinem der vorstehenden Ansprüche, dadurch gekennzeichnet, daß das Lösungsmittel unter Benzol, Toluol, Chlorbenzol, Hexan, Cyclohexan, Methylenchlorid, 1,2-Dichlorethan und Diphenylether ausgewählt wird.

5. Verfahren nach irgendeinem der vorstehenden Ansprüche, dadurch gekennzeichnet, daß das Lösungsmittel mindestens 10 Vol.-% des Reaktionsmediums darstellt.

6. Verfahren nach Anspruch 5, dadurch gekennzeichnet, daß das Lösungsmittel 30 bis einschließlich 90 Vol.-% des Reaktionsmediums darstellt.

7. Verfahren nach irgendeinem der vorstehenden Ansprüche, dadurch gekennzeichnet, daß die Konzentration an Iridium in dem Reaktionsmedium zwischen 10⁻³ und einschließlich 10⁻¹ Mol/l beträgt.

8. Verfahren nach irgendeinem der vorstehenden Ansprüche, dadurch gekennzeichnet, daß die Reaktionstemperatur zwischen 100 und 240 °C und vorzugsweise zwischen 160 und 190 °C beträgt.

9. Verfahren nach irgendeinem der vorstehenden Ansprüche, dadurch gekennzeichnet, daß der Partialdruck des Kohlenmonoxids zwischen 1 und 250 bar und vorzugsweise zwischen 2 und 100 bar beträgt.

10. Verfahren nach irgendeinem der vorstehenden Ansprüche, dadurch gekennzeichnet, daß das atomare Verhältnis I/Ir niedriger als 10 beträgt.

11. Verfahren nach irgendeinem der vorstehenden Ansprüche, dadurch gekennzeichnet, daß das atomare Verhältnis I/Ir höher als oder gleich 0,1 beträgt.

12. Verfahren nach irgendeinem der vorstehenden Ansprüche, dadurch gekennzeichnet, daß das atomare Verhältnis I/Ir zwischen 1 und einschließlich 5 liegt.

13. Verfahren nach irgendeinem der vorstehenden Ansprüche, dadurch gekennzeichnet, daß man als Iod-Promotor HI verwendet.
